# EUROPEAN PATENT APPLICATION

(11) **EP 1 669 031 A1**
(43) Date of publication of application: **14.06.2006**
(21) Application number: 04106451.0
(22) Date of filing: 10.12.2004
(51) Int. Cl.: A61B 8/14, A61B 5/0456

(54) **Method of selecting part of a run of echocardiography images**

(71) Applicant: AGFA-GEVAERT, 2640 Mortsel (BE)
(72) Inventor: Van Goubergen, Herman AGFA-GEVAERT, 2640 Mortsel (BE)

(57) **Abstract**

In a system wherein an electrocardiogram corresponding with a run of images is acquired and displayed simultaneously with the run of echocardiography images, data are deduced from the digital signal representation of the displayed electrocardiogram and these data are used to identify said part of the run of images.

## Description

### FIELD OF THE INVENTION

The present invention relates to a method of selecting part of a sequence of images (also called 'run') in an echocardiography application. The method is applicable to echocardiography applications in which a corresponding electrocardiogram image is displayed simultaneously with a run of images.

The selected part of the run can be converted to DICOM format, archived, displayed etc.

### BACKGROUND OF THE INVENTION

Echocardiography is a non-invasive procedure used to examine the heart and potentially diagnose problems.

Diagnostic ultrasound employs pulsed, high frequency (>20,000 Hz) sound waves that are reflected back from body tissues and processed by the ultrasound machine to create characteristic images. Defined bursts of ultrasound waves are emitted from the transducer, which also acts as a receiver. Ultrasound can be aimed in a specific direction and obeys the laws of geometric optics with regard to reflection, transmission and refraction. When an ultrasound wave meets an interface of differing echogenicity, the wave is reflected, refracted and absorbed. Only reflected sound waves (echos) can be sensed by the transducer and processed. The transducer acts as a receiver over 99% of the time.

Commonly the moving echocardiography image which is displayed on the screen of the echocardiography modality is digitized during patient examination. It is also common practice to convert part of this run into DICOM format and to archive the DICOM images.

Depending on the work flow a number of different modes for capturing part of the run of images can be defined. These modes specify when to start and when to stop capturing video data. Examples are : capturing a number of images corresponding with a number of heartbeats (e.g. three) after a button is activated or capturing continuously until a button is activated, then store the last 2 complete heartbeats, etc.

In these workflows it is often required that a stored run or part of a run of images (for example two heartbeats) can be played over and over again in a loop thereby creating the illusion of a continuously beating heart on the display screen. Sudden jumps in the displayed stream of images during play back are to be avoided.

Conventionally an external trigger provided by the echocardiography modality which generates an electronic pulse at a specific phase of a patient's heartbeat cycle is used as a signal to start or stop digitizing a video stream or to determine which part of a digitized run will be stored.

This conventional approach has the following drawbacks:
- a physical connection is required between the digitizing equipment and the echocardiography modality. Such a need may complicate installation and may introduce electric shielding and safety issues.
- moreover, not all modalities provide such a synchronizing signal or do not provide it externally so that unscrewing of apparatus parts may be required.

Even if the synchronizing signal is available external to the modality, then it is likely that different connection kits are required for different types of modalities since connector types and signal levels may be dedicated to the specific type of modality.

It is an aspect of the present invention to provide a method of selecting a part of a run of echocardiography images that overcomes the drawbacks of the prior art.

### SUMMARY OF THE INVENTION

The above-mentioned aspects are realized by a method having the specific features set out in claim 1. Specific features for preferred embodiments of the invention are set out in the dependent claims.

Further advantages and embodiments of the present invention will become apparent from the following description and drawing.

According to the present invention data which are used to identify a part of a run of images are deduced from the digital image of the electrocardiogram data of the patient which is simultaneously displayed with the echocardiography moving image.

The present invention as well as preferred embodiments thereof will be described below with reference to the accompanying drawing.

### DETAILED DESCRIPTION OF THE DRAWINGS

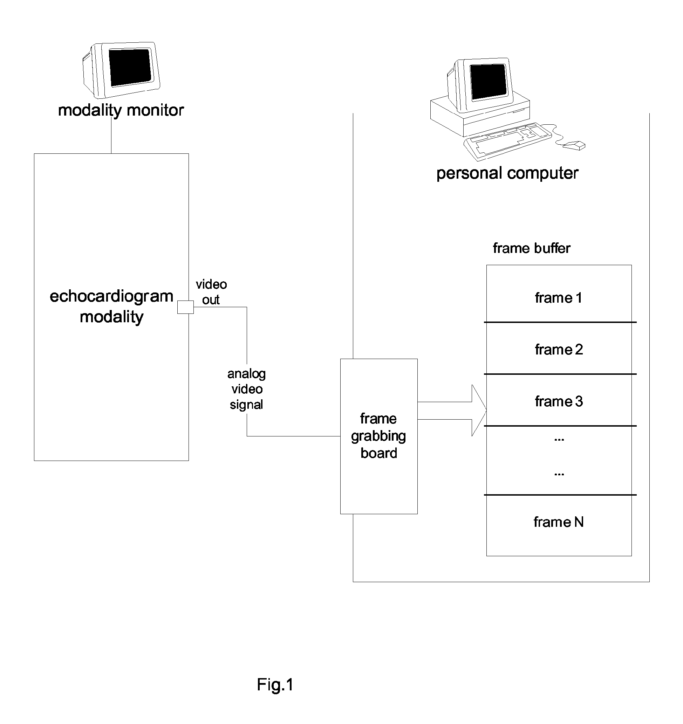

Figure 1 illustrates an application in which the present invention is applicable.

### DETAILED DESCRIPTION OF THE INVENTION

Figure 1 shows an echocardiography modality and an associated monitor on which an electrocardiogram can be displayed.
The invention is applicable to echocardiography modalities which are able to display in addition to the actual echocardiogram of a patient the patient's electrocardiogram.

The echocardiography modality has a video output connector which can be connected to the input of a frame grabbing board coupled to a personal computer. The frame grabber captures and digitizes successive frames from the analog video output signal of the echocardiography modality. The output of the frame grabbing board, being a number of digitized frame images, denoted frame 1 ... frame N, can be stored as bit maps in a frame buffer.

In the context of the present invention each of these frame images comprises at least an image of a run of echocardiography images and a corresponding part of the ECG plot of the heart that is examined.

According to the present invention, the signal pertaining to the ECG image which is displayed simultaneously with the corresponding echocardiography images is separated from the composite image signal (echo data and electrocardiogram data) and is analyzed.

The ECG signal or at least data deduced from this signal is subsequently used as a synchronising signal in the process of selection of a part of a run of echocardiography image frames.

Commonly the echocardiography image is at the center of the display screen and the ECG plot is displayed near the top or bottom of the screen so that the plot doesn't hide too much of the ultrasound image details.
The ECG plot is commonly drawn in a contrasting colour, (often green) using the echocardiography image as a background.
The ECG plot is usually not displayed in a separate, dedicated window with a neutral background, which would have made it easier to separate ECG from echo data.
The ECG plot scrolls horizontally from right to left, so the most recent ECG data is written at the right edge of the plot, while the older data is shifted to the left.
Modalities usually provide ways for the operator to :
- enable/disable display of the ECG plot,
- control of the location on the screen where the ECG is plotted (top/bottom)
- change the vertical scale of the ECG plot (the displayed amplitude)
- change the horizontal scale of the ECG plot (being the time scale which determines how many seconds of the ECG are visible on the screen at any one time and also influences the scroll speed).

Certain information about the appearance of the ECG plot on a specific modality can be given at installation time, however most of the information will have to be extracted from the images when a run of images has been captured.

For this purpose a stream of video data have to be captured (more data than are intended to be archived), analysed to get synchronisation information from the ECG image. Next, in accordance with a capturing mode selected by the user (for example the need to store images corresponding with two heart beats) it has to be decided which part of the run of images will be discarded and which part will be upheld and e.g. converted to DICOM and e.g. archived.

The process of getting the information about the patient's ECG is built up in the following stages:
(a) identify color and general location of the ECG plotted image
(b) identify precise location of ECG plot in a displayed image
(c) filter out ECG plot baseline
(d) find scroll speed of ECG plot
(e) reconstruct static ECG plot
(f) process ECG data
(g) determine heartbeat period
(h) determine position of a specific phase within heartbeat period.

(a) First the location of the ECG plot in composed image of ECG plot and echocardiography image is determined.

As it is unlikely that the ECG plot color or general location on the screen will change often on a given modality, this first step will commonly only be performed when installing or configuring the image capture system.

The installer should indicate roughly what colour the ECG plot is (green, cyan, amber,...), and (in case this colour is used in any other part of the screen) where the ECG plot is located (top half, bottom half,...).

The digital signal representation (bit map) of a frame image is considered and within the ECG part of the frame image color clustering is performed. This will result in a number of distinct color clusters. The cluster with the mean color closest to the color of the ECG plot indicated by the installer is selected. The mean color of the cluster is then taken as the ECG-color, and the dimensions (in RGB space) of that cluster are stored. From this moment on, to test whether a pixel is part of the ECG plot, it is verified whether the pixel's RGB values lie within the cluster.

This is the most primitive way of identifying the ECG plot color. If a more interactive user interface is available it would be preferable to display the digitized image, have the installer indicate the ECG plot with a pointing device and use that input to determine the ECG-color.

(b) In the next step the exact location of the ECG plot on the display screen is identified.

The location of the ECG plot on the screen is determined by analyzing a captured run of images.

To find the position and size of a rectangle which encloses the ECG plot a limited number of frame images is selected. These frame images are preferably spaced equally through the captured run. For each frame image a rectangular area is calculated within which pixels with the ECG-color are present. Then all these rectangular areas are combined to end up with one rectangle which encloses them all. This area is called the ECG-rectangle.

A number of frame images throughout the run is looked at to ensure that the height of the ECG plot is not underestimated. If a single image would have been used which contains ECG data during a period where the patient's ECG amplitude was low, the rectangle would not be high enough. This is avoided by evaluating a number of images.

(c)In this step the baseline of the ECG plot is filtered out.

Sometimes the displayed ECG plot has a horizontal line running through it (at the 0 level). Though the line is drawn in the ECG color, it isn't part of the patient's ECG data and, if it is present, it needs to be filtered out. To detect the baseline(s) (thickness could be more than one pixel) a frame image is taken from the digitized run. Within the ECG-rectangle in this image, horizontal lines drawn in ECG-color which run across the complete width (or at least >98%) of that rectangle are looked for.
The vertical position of such lines is determined.
Then this procedure is repeated for another frame image from the run.
The marked horizontal line(s) common to both images are considered plot baseline(s) and their location can be stored.

Now all the elements required to detect the pixels which are part of the ECG plot are determined: pixels of the ECG plot are located within the ECG-rectangle, they have a color which is close to the ECG-color indicated at installation, and pixels on the ECG-baseline are not part of the ECG plot.

(d) In the following step the scroll speed of the ECG plot is determined.

The scroll speed can be expressed in number of pixels scrolled to the left per displayed image.

In the following explanation it is assumed that a run of captured images contains N images, the last captured image will be referred to as frame image N, the next to last as frame image N-1 etc.

The procedure is as follows.
Start at last frame image N of the run. Locate the ECG-plot pixels of that image.
Repeat with image N-4.
Superpose the ECG-data from both images and determine the degree of match between these images, e.g. through pixel correlation.
Repeat this procedure but this time shift the ECG data from the N-4 image one pixel to the left. Determine the degree of match between the images.
Repeat again with a 2 pixel shift and so on.
At the end, select the pixel shift which produced the best match. Now it is known how many pixels the ECG shifted to the left between image N-4 and N.

This comparison procedure is repeated with image N and image N-8 to verify the measurement: this pixel shift should be about twice as large.

To improve the accuracy, an extra step is performed. The measured pixel shift is used to calculate how many images are needed to go back in time to shift the ECG data across a large distance (half the width of the ECG-rectangle). This number of images will be called D. The comparison procedure is repeated for image N and image N-D. A pixel shift of (about) half the width of the ECG rectangle should be found.

This should confirm earlier measurements and should improve accuracy: the speed is not necessarily an integer number of pixels and if all calculations would have been based on the comparison between image N and N-4, the inaccuracy might accumulate for large image distances.

It will be clear that the above numbers N-2, N-4 etc. are not meant to be limitative for the present invention and that other numbers are also applicable.

(e) In this step a static ECG plot is reconstructed.

The static ECG plot is reconstructed by pasting together the ECG-data within the ECG rectangle pertaining to image N with the ECG-data within the ECG rectangle pertaining to image N-D. These two ECG rectangles overlap by a distance of half a rectangle-width.
This overlap is used to verify that the images are correctly lined up.

It might be necessary to shift one rectangle a few pixels left or right to get a perfect match in case the scroll-speed is was not entirely accurate.
Then this procedure is repeated with image N-2D and so on until a complete and static representation of the whole ECG is obtained which scrolled across the screen while image frames were being captured. This results in a very wide bitmap.

Now for each column of this bitmap the topmost ECG-pixel is searched for. In this way the bitmap is converted into an array of ECG amplitude values.
These values are similar to what would have been obtained when the original ECG signal would have been sampled.

The ECG sampling frequency can now also be calculated.
Since it is known how many new ECG samples have scrolled on to the screen per image (the scroll speed has been calculated) and it is also known from the video parameters used to digitize the video signal, how many images are displayed per second.
This provides the exact time scale of the ECG plot.

(f) In a subsequent step the ECG data are processed in case of occasional gaps in the signal.

Sometimes there are gaps in the ECG plot, i.e. in some places the plot is not a continuous line but a series of disjoint dots. These gaps can have several origins:
- they can be interlacing artefacts,
- they can be caused by the horizontal scaling of the ECG plot by the modality
- they can be caused by a sudden high peak or valley in the ECG plot which extends beyond the screen height (the same effect can occur when the height of the ECG plot is underestimated by the algorithm to calculate the ECG rectangle)
- they can be caused in zones where the ECG plot coincides with the baseline which was filtered out.

For the ECG-amplitude array that has been constructed, this means that some array entries will be empty. These unknown values are filled in by values obtained by interpolating between the known neighbouring ECG amplitudes.

For detecting peaks in the ECG the absolute amplitude is not interesting but the differences between successive amplitudes is important. Therefore the ECG array is converted into a delta array delta[i] = ECG[i] - ECG[i-1].

(g) In the following step the heartbeat period is determined.

In general the structure of one heartbeat and the next are fairly similar.
A block of consecutive entries at the end of the delta array is taken and compared to an equally wide block which is approximately 1 average normal heartbeat earlier in the delta array and calculate how well the blocks match, e.g. by a correlation procedure.
This procedure is repeated letting the distance between the 2 blocks range from minimal to maximal possible heartbeat period.
Then the block distance is selected which produces the best match. That distance corresponds to the period of the patient's heartbeat.

(In the previous procedure the width of the block corresponds to the maximal possible heartbeat period.)

To confirm the measurement the procedure is repeated in earlier sections of the delta array. If widely varying measurements are obtained (difference between consecutive beats larger than 1/10 beat) it is concluded that ECG sync detection failed. If they are similar, we calculate the mean period. This mean value is the patient's heartbeat period.

(h) Finally the position of a specific phase within the heartbeat period is determined.

To determine the position of a specific phase within the heartbeat period the R-peak is used. At the position of the R peak the ECG amplitude peaks high and is followed by a very steep drop.

To find the position of the R peak, the last section of the delta array which is one heartbeat wide is considered and the largest falling edge within that section is selected. The start of that falling edge corresponds to the R peak.

The previous R peak is found by moving back one heartbeat in the delta array and then again fine-tuning by comparing a block of the delta array centered around the last R peak with the delta values near the probable location of the penultimate R peak and selecting the position where the blocks match best. (here we use block width of 1/8 heartbeat). The procedure is repeated to detect the R peak before that on and so on.

As the scroll speed of the ECG is already known, one can calculate back from R-peak location in the delta array to the number of the image in which that R-peak was just visible at the right edge of the ECG plot.

Using this info it can be decided which part of the run of images to discard and which part to store depending on the capturing mode selected by the user.

Having described in detail preferred embodiments of the current invention, it will now be apparent to those skilled in the art that numerous modifications can be made therein without departing from the scope of the invention as defined in the appending claims.

## Claims

1. Method of selecting part of a run of echocardiography images in a system wherein an electrocardiogram corresponding with said run of images is acquired and displayed simultaneously with said run of echocardiography images,
**characterised in that** data are deduced from the digital signal representation of the displayed electrocardiogram, said data being used to identify said part of the run of images.

2. Method according to claim 1 wherein for a given capturing mode the start of said part of a run of echocardiography images is determined by means of said data deduced from the corresponding ECG.

3. Method according to claim 1 wherein a video output of a echocardiography image acquisition apparatus is subjected to a frame grabbing operation resulting in a number of frame images comprising an echocardiography image and a plot of a corresponding electrocardiogram (ECG) image and
wherein said digital signal representation of the ECG image is extracted from at least one of said frame images.

4. Method according to claim 3 wherein said signal representation of an ECG image is extracted by performing a colour clustering operation on at least one frame image and by deciding for each pixel of said frame image(s) whether it is part of a colour cluster pertaining to the image of said ECG.

5. A method according to claim 3 wherein a base line is filtered out of said signal representation of said ECG image.

6. A method according to claim 3 wherein the scroll speed of the ECG image is deduced from a sequence of said frame images.

7. A method according to claim 3 wherein a static ECG plot is reconstructed from digital signal representations of ECG images extracted from at least two image frames taking into account said scroll speed.

8. A method according to claim 7 wherein missing values in the signal representation of the ECG are filled with values obtained by interpolating between neighbouring ECG signal values.

9. A method according to claim 3 wherein the heartbeat period of the heart that is evaluated in the echocardiography application is obtained by
- converting the digital signal representation of the ECG image into a delta array whereby delta[i]=ECG[i] - ECG[i-1], whereby ECG[] represent ECG signal values,
- evaluating match between blocks of consecutive entries in said delta array spaced apart by a distance which is equal to an approximation of the heart beat period,
- selecting the period resulting in optimal match.

10. A method according to claim 3 wherein the position of a specific phase within the ECG signal representation corresponding with a heartbeat period is determined.

11. A method according to claim 10 wherein said position of a specific phase is the position of the R peak in the ECG signal.

12. A method according to claim 10 wherein said position of a specific phase determines the start of said part of a run of frames.
